# EUROPEAN PATENT SPECIFICATION

(11) **EP 1 056 443 B1**
(45) Date of publication and mention of the grant of the patent: **06.05.2004**
(21) Application number: 99936027.4
(22) Date of filing: 25.02.1999
(51) Int. Cl.: A61K 9/08, A61K 9/10, A61K 31/485, A61K 47/44, A61K 47/02, A61K 47/12

(54) **BUTORPHANOL SUSTAINED RELEASE FORMULATIONS**
BUTORPHANOL ENTHALTENDE FORMULIERUNGEN MIT VERZÖGERTER FREISETZUNG
FORMULATIONS DE BUTORPHANOL A LIBERATION PROLONGEE

(30) Priority: 25.02.1998 US 30217
(43) Date of publication of application: 06.12.2000
(73) Proprietor: ABBOTT LABORATORIES, Abbott Park, IL 60064-6050 (US)
(72) Inventor: CHANG, Hung-Chih, Gurnee, IL 60031 (US); LI, Lukchiu, Vernon Hills, IL 60061 (US); TIAN, Youqui, Mundelein, IL 60060 (US)
(74) Representative: Modiano, Guido, Dr.-Ing.
(86) International application number: PCT/US1999/004113
(87) International publication number: WO 1999/043300

(56) References cited:
- EP-A- 0 300 806
- WO-A-94/14426
- US-A- 4 338 324
- US-A- 4 464 378
- PFEFFER M. ET AL: "Pharmacokinetics of Subcutaneous and Intramuscular Butorphanol in Dogs" J. OF PHARM. SCIENCES, vol. 69, no. 7, 1980, pages 801-803, XP002109395
- CHANG, H.-C. ET AL: "Parenteral sustained-release dosage forms of butorphanol for dogs" INT. J. PHARM. (1999), 176(2), 147-156 , XP002109396

## Description

### Technical Field of the invention

The field of this invention is long-term pain management. More particularly, the present invention relates to controlled release formulations of the opioid analgesic, butorphanol, and the use of such formulations for pain management over periods of time ranging from 12 to 24 hours.

### Background of the Invention

Butorphanol is a synthetic opioid agonist-antagonist that is highly effective for the treatment of both chronic and acute pain. Parenterally administered butorphanol is more potent than morphine and most other morphine analogs. Butorphanol is metabolized in the liver and excreted by the kidney. The elimination of butorphanol and its metabolites is rapid and the duration of analgesia is usually in the range of three to four hours, with maximal analgesia obtained one-half to one hour following parenteral administration. Butorphanol can be used to treat acute surgical pain, severe post-operative pain and chronic pain. The drug has been shown to be an effective pain management therapy in treating pain associated with numerous types of surgery, bums and kidney stones.

Parenteral formulations of butorphanol and the use of parenteral butorphanol for the relief of acute and chronic pain are known in the art (See, e.g., United States Patent No. 3,819,635). A parenteral formulation of butorphanol is commercially available under the name STADOL® from Bristol-Meyers Laboratories, Inc. 2-4 mg of that formulation are typically injected intramuscularly for the treatment of post-operative pain. Typically, dosing interval ranges from about three to about four hours are needed to sustain analgesia.

A sustained release formulation of butorphanol encapsulated in phospholipid vesicles or lipisomes is disclosed in European Patent Application 0300806A1. In accordance with that disclosure, butorphanol tartrate in phosphate buffered saline is encapsulated with lipid films of distearoylphosphatidylcholine and cholesterol. Effective levels of analgesia ranging from 12 to 24 hours can be achieved by administering such lipisome encapsulated butorphanol.

South African Patent Application 91/4549 discloses sustained release pharmaceutical formulations and the use of those formulations in delivering therapeutic agents over periods of time of from about 12 to 24 hours. Formulations disclosed in that South African patent application are microspheres of between 5 to 300 micrometers that contain at least one pharmaceutically active substance contained in a spherical structure formed by at least one pharmacologically inactive carrier substance. The carrier substance is naturally present in the organism to be treated and is stable in the solid state up to a temperature of at least 60°C. Exemplary such carrier substances are coprosterol, glycocholic acid, cholesterol and cholesterol esters. Pharmaceutically active substances that can be administered using such microspheres are tranquilizers, anti-emetics, vasodialators, antihistiminics, steroids and analgesics.

Existing sustained release formulations are costly and require multi-step manufacturing procedures. There continues to be a need in the art for simple, inexpensive formulations of butorphanol that provide a controlled, sustained release of the drug over long periods of time.

### Brief Summary of the Invention

In one aspect, the present invention provides a pharmaceutical composition for the controlled release of butorphanol. The composition contains an effective therapeutic amount of butorphanol free base suspended in an aqueous buffer having a pH less than 7.0. In a preferred embodiment, the pH is from 6.2 to 6.5 and, more preferably from 6.2 to 6.3. The composition is preferably in the form of microparticles having an average diameter of from 5 to 25 microns. Preferably, the sizes of the microparticles are from 5 to 15 microns.

The aqueous composition can contain a wide range of effective butorphanol concentrations. A particularly suitable concentration range is from about 4 to about 10 mg/ml and, preferably from 5 to 7.5 mg/ml.

An aqueous composition of this invention can further include an effective amount of a non-ionic solubilizing agent. Exemplary and preferred such solubilizing agents are polyoxyethylene sorbitan fatty acid esters such as polysorbate 80. The composition can still further include an effective amount of a preservative. A preferred preservative is an alkylparaben such as methylparaben or propylparaben.

In another aspect, the present invention provides a pharmaceutical composition for the controlled release of butorphanol, which composition includes an effective therapeutic amount of a butorphanol salt suspended in oil. A preferred butorphanol salt for use in such a composition is butorphanol tartrate.

In a preferred embodiment, butorphanol is present in such a composition in a concentration range of from 5 to 30 mg/ml and, more preferably from 10 to 20 mg/ml. Any GRAS oil known in the pharmaceutical art can be used in the composition. Exemplary and preferred such oils are cottonseed oil, corn oil, peanut oil, sesame oil and soybean oil. Soybean oil is most preferred.

The oil-based composition can further include an effective amount of a suspending agent or emulsifier. A preferred suspending agent is a sorbitan fatty acid ester such as a sorbitan mono-, di- or tri-laurate, a sorbitan mono-, di- or tri-oleate, a sorbitan mono-, di- or tri-palmitate, or a sorbitan mono-, di- or tri-stearate. An especialy preferred sorbitan fatty acid ester is span 85.

Either the aqueous- or oil-based composition can be used to provide effective long-term pain management in patients. The injection of an effective therapeutic amount of either composition provides effective pain management over a period of time of 12 to 24 hours and preferably over a time period of 18 to 24 hours. Effective long-term pain management is accomplished by injecting an amount of either composition sufficient to maintain plasma butorphanol concentration at a level of between 20 and 100 ng/ml.

### Detailed Description of the Invention

### 1. The Invention

Pharmaceutical compositions for the controlled, sustained release of an opioid analgesic are provided. The composition can be formulated either as an aqueous- or an oil based formulation. Injection of a composition of this invention has use in providing long-term pain management in patients in need of such therapy.

### II. Aqueous Formulation

In one embodiment, a composition of this invention is an aqueous suspension of butorphanol. Butorphanol is an art recognized pain relieving agent that belongs to the class of analgesics known as synthetic opioid agonists-antagonists. Butorphanol can exist either as a free base or as a salt. For use in an aqueous formulation of the present invention, the free base form is used. As is known in the art, the free base can be made by titrating a butorphanol salt (e.g., butorphanol tartrate) with an alkali metal salt such as NaOH.

The composition contains an effective therapeutic amount of butorphanol free base suspended in a buffered aqueous medium. The pK, of butorphanol is 8.6. The solubility of butorphanol is inversely proportional to pH over the pH range of 6.0 to 8.5. By way of example, at a pH of: 6.25, 60% of butorphanol is in solution whereas at a pH of 7.0. only 5% of the drug is in solution. As shown hereinafter in the Examples, however, formulations at either pH release virutally 100% of the drug over 48 hours. The initial rate of release of the drug is somewhat higher at low pH's. A preferred pH for an aqueous composition of the present invention is between 6.0 and 7.5. More preferably, the pH is from 6.25 to 7.0. pH of the composition is maintained at the desired level through the use of pharmaceutically acceptable buffers such as sodium citrate or sodium phosphate.

The aqueous composition can be made to contain a wide range of effective butorphanol concentrations. The only limit on butorphanol levels is the solubility of the drug in the composition. A particularly suitable concentration range in a composition having a pH of between 6.0 and 7.5 is from 4 to 20 mg/ml. More preferably, the concentration of butorphanol is from 5 to 15 mg/ml.

The aqueous composition can include substances other than buffers and butorphanol. In one embodiment, the composition further includes an effective amount of a non-ionic solubilizing agent. Exemplary and preferred such solubilizing agents are polyoxyethylene sorbitan fatty acid esters such as polysorbate 80. Suitable levels of such solubilizing agents are well known in the an and depend *inter alia* on the particular solubilizer used. When the solubilizing agent is polysorbate 80, a suitable effective amount is from .0.5 to .2.0 weight percent.

The composition can still further include an effective amount of a preservative. Suitable preservatives for use in pharmaceutical compositions are well known in the art (See, e.g., Handbook of Pharmaceutical Excipients, published by the American PharmaceuticalAssociation and the the Pharmaceutical Society of Great Britain). An exemplary and preferred preservative is an alkylparaben such as methylparaben or propylparaben. The preservative is typically present in the composition in a concentration range of from 0.01 to 0.4 mg/ml and, more preferably from 0.02 to 0.2 mg/ml.

The composition can be formulated as microparticles by passing the composition through a microfluidizer as is well known in the art. Preferred microparticles have an average diameter of from 5 to 25 microns. More preferably, the size of the microparticles is from 5 to 45 microns. A detailed description of the preparation and contolled release characteristics of aqueous butorphanol suspensions can be found hereinafter in the Examples.

### III. Oil Formulation

A controlled release butorphanol composition can also be in the form of an oil suspension or an oil/water emulsion. In accordance with this embodiment, an effective therapeutic amount of a butorphanol salt is suspended in a pharmaceutically acceptable oil. A preferred butorphanol salt for use in such a composition is butorphanol tartrate.

In a preferred embodiment, butorphanol is present in such a composition in a concentration range of from 5 to 30 mg/ml and, more preferably from 10 to 20 mg/ml. Any GRAS oil known in the pharmaceutical art can be used in the composition. Exemplary and preferred such oils are cottonseed oil, corn oil, peanut oil, sesame oil and soybean oil. Soybean oil is most preferred.

The oil-based composition can further include an effective amount of a suspending agent. A preferred such suspending agent is a sorbitan fatty acid hexitan ester such as a sorbitan mono-, di- or tri-laurate, a sorbitan mono-, di- or tri-oleate, a sorbitan mono-, di- or tri-palmitate, or a sorbitan mono-, di- or tri-stearate. An especialy preferred sorbitan fatty acid ester is commercially available as Span 85. The suspending agent is typically present in a concentration of from 0.5 percent (w/v) to 2.0 percent (w/v).

### IV. Process of Pain Management

Either the aqueous- or oil-based composition can be used to provide effective long-term pain management in patients. The injection of an effective therapeutic amount of either composition provides effective pain management over a period of time of 12 to 24 hours and preferably over a time period of 18 to 24 hours. Effective long-term pain management is accomplished by injecting an amount of either composition sufficient to maintain plasma butorphanol concentration at a level of between 20 and 100 ng/ml.

The Examples that follow illustrate particular embodiments of the present invention.

### EXAMPLE 1: Preparation of Butorphanol Formulations

### A. Preparation of the Aqueous Suspensions

In the preparation of the aqueous suspensions, butorphanol tartrate was used as the starting material and the free base was produced *in-situ* by neutralizing the salt with a base solution. Two aqueous suspensions have been prepared and evaluated.
i. 5 mg/mL Aqueous Suspension

**Table 1.**

| Formulation of the 5 mg/mL Aqueous Suspension | |
|---|---|
| Ingredient | Amount |
| Butorphanol Tartrate | 1.0 g |
| Tween 80 | 1.0 g |
| Phosphate Buffer (0.2 M) | 140.0g |
| Methylparaben | 40 mg |
| Propylparaben | 4.0 mg |
| 1N NaOH | 24 g |
| Water for Injection | q.s. to 200 mL |

In brief, 40 mg methylparaben, 4 mg propylparaben, and 1 g Tween 80 were dissolved in 140 g phosphate buffer by heating the mixture to ∼55°C. One gram of butorphanol tartrate was dissolved into the solution. To produce butorphanol free base, sodium hydroxide (NaOH) was then added dropwise into the solution mixture containing the drug under agitation, using the Silverson high-shear mixer at 7,000 rpm for five minutes. Water for injection was added to make a final volume of 200 mL. The pH of the suspension was 6.25. The resulting suspension was passed through the Microfluidizer 110Y (Microfluidics, Newton, MA) for 10 cycles with the 75-µm interaction chamber and the 200 µm back-pressure module at 12,000 psi. The drug particle size has been estimated, by visual examination under an optical microscope, to be in the range of 5-10 µm.
ii. 10 mg/mL Aqueous Suspension

**Table 2.**

| Formulation of the 10 mg/mL Aqueous Suspension | |
|---|---|
| Ingredient | Amount |
| Butorphanol Tartrate | 2.0 g |
| Tween 80 | 1.0 g |
| Citrate Buffer (0.2 M) | 120.0g |
| 1N NaOH | 61 g |
| 0.1N NaOH | 5.0 g |
| Water for Injection | q.s. to 200 mL |

In this formulation, preparation procedures similar to those for the 5 mg/mL suspension were employed. One gram of Tween 80™ was dissolved in 120 g citrate buffer by heating the mixture to ∼55°C. Then, 1 g of butorphanol tartrate was dissolved into this solution. To produce butorphanol free base, sodium hydroxide was added dropwise into the solution mixture containing the drug under agitation, using the Silverson high-shear mixer at 7,000 rpm for five minutes. Water for injection was added to make a final volume of 200 mL. In the resulting suspension, the pH was 7.00 and the particle size has been estimated, by visual examination under an optical microscope, to be between 30 and 40 µm.

### B. Preparation of the Oil Suspension

Two oil suspensions were prepared and evaluated (Tables 3 and 4).

**Table 3.**

| Formulation of the 20 mg/mL Oil Suspension | |
|---|---|
| Ingredient | Amount |
| Butorphanol Tartrate | 2 g |
| Span 85 | 1 g |
| Soybean Oil | q.s. to 100 mL |

**Table 4.**

| Formulation of the 10 mg/mL Oil suspension | |
|---|---|
| Ingredient | Amount |
| Butorphanol Tartrate | 1 g |
| Span 85 | 1 g |
| Soybean Oil | q.s. to 100 mL |

To prepare the oil suspension, a predetermined amount of spray-dried butorphanol tartrate (2-10 µm) was placed in a beaker and a predetermined amount of a suspending agent (Span 85) was added and mixed with the drug until a smooth paste was formed. Oil was subsequently added in small portions with continuous stirring until the required volume was achieved. The final product was mixed using a Silverson® high-shear mixer for five minutes at 5,000 rpm and was stored at room temperature for further evaluation.

### C. Analysis of the Butorphanol Content in Formulations

i. Aqueous Suspension
   The drug content of selected butorphanol/aqueous suspensions was analyzed by HPLC assay: One mL of the aqueous suspension was pipetted into a 100mL volumetric flask. Ten mL of 1N sulfuric acid was subsequently added to dissolve the drug. The volume was then made up to 100 mL with water and directly injected onto the HPLC. In order to determine the drug content in the solution phase, 3 mL of the suspension was filtered through a 0.22 µm filter. The filtrate was then directly injected onto the HPLC as described above.
   The HPLC system consisted of a Model SIL-9A autoinjector (Shimadzu®), a Model SPD-6A UV-spectrophotometer (Shimadzu®), and a Model CR501 integrator (Shimadzu®). The stationary phase was a µBondapak™ Phenyl column (3.9 x 300 mm, Waters, USA). The mobile phase was prepared by mixing 1 L 0.05M ammonium acetate and 335 mL acetonitrile, with pH adjustment to 4.1 using glacial acetic acid. The chromatogram was monitored by UV detection at the maximum wavelength of 280 nm, with a sensitivity setting of 0.08 AUFS. The injection volume was 100 µL and the flow rate was 2 mL/min.
ii. Oil Suspension
   The drug content of selected butorphanol tartrate/oil suspensions was analyzed by HPLC assay: One mL of the oil suspension was pipetted and placed in a centrifuge tube. Ten mL 1N sulfuric acid was added. The tubes were shaken using a Burell wrist-action shaker for a period of 15 minutes and then centrifuged for five minutes at 3000 rpm. The aqueous phase was separated from the oil phase and further diluted before being injected into the colum. In order to determine the amount of drug in the oil-solution phase, 3 mL of the oil suspension wsa filtered through a 0.22 µm filter. One mL of this filtrate was pipetted into a centrifuge tube and 10mL 1N sulfuric acid was added to the tube, which was subsequently shaken and centrifuged as described above. The aqueous solution was sampled and injected directly onto the HPLC.

### D. Evaluation of Butorphanol Release

A predetermined amount of aqueous or oil suspension was transferred, in duplicate, to dialysis tubing (Spectra®)/Por2, MWCO 12,000-14,000) and the tubing was placed in 100mL of 0.1M phosphate buffer (pH 7.4) being used as dialyzate. Agitation was achieved using a magnetic stirrer at a speed of 600 rpm. At a predetermined sampling time interval, 1 mL of the dialyzate was withdrawn for content assay using the HPLC method as described above. To maintain the drug-release study under sink conditions, the residual dialyzate was discarded and replaced with fresh medium at each sampling time point.

The drug-release profiles for the 10 mg/mL and 20 mg/mL oil suspensions were similar. The total drug release was over 80% after 48 hours. The data also show that the drug dissolution rate did not slow down with a higher percentage of oil surrounding the drug particles. The drug-release profiles of butorphanol free base from aqueous suspensions with two different pHs and drug-particle sizes showed that the total cumulative drug releases from these two aqueous formulations were close to 100% after 48 hours. However, drug release in the first 24 hours was much faster in the 5 mg/mL suspension (pH 6.25) compared to that of the 10 mg/mL (pH 7.00). The faster release rate may be attributed to the higher solubility of the drug at this lower pH and the smaller drug-particle size.

### EXAMPLE 2: In-vivo Evaluations of Formulations

### A. Animal Study Design

Fifteen beagle dogs were obtained from the established Abbott-Drug Analysis Colony. The dogs were randomly assigned to one of the following five groups; each group contained three animals.

**Table 5.**

| Formulations Tested in Dogs | | |
|---|---|---|
| Formulation | Concentration | Description |
| A | 5 mg/mL | Aqueous Suspension |
| B | 10 mg/mL | Aqueous Suspension |
| C | 10 mg/mL | Oil Suspension |
| D | 20 mg/mL | Oil Suspension |
| E | 2 mg/mL | Torbugesic-SA Injection |

The dogs received a 2 mg/kg subcutaneous dose of either one of the experimental suspension formulations or the reference immediate-release injection. Heparinized blood samples were obtained from a jugular vein of each dog prior to dosing and 0.25, 0.5, 1, 2, 4, 6, 9, 12, 15, 24, 48, and 72 hours after dosing. The plasma was separated from the red cells by centrifugation (2500 rpm/10 minutes, 4°C) and frozen -20°C for subsequent analysis.

### B. Drug Analysis for Animal Study

Butorphanol and an internal standard were separated from the plasma matrix using liquid-liquid extraction with chloroform under alkaline conditions. Briefly, 0.5 mL plasma was mixed with 0.1 mL internal standard 1.0 mL 1N NaOH, and 7 mL chloroform. The samples were vortexed for 20 seconds, followed by centrifugation. The upper aqueous layer was aspirated to waste. The organic layer was transferred to a conical centrifuge tube and evaporated to dryness with a gently stream of dry air over low heat (∼35°C). The samples were reconstituted in 0.2 mL acetonitrile:water (3:7, by volume). Butorphanol plasma standards were analyzed simultaneously with the samples.

Butorphanol and the internal standard were separated from the co-extracted plasma contaminants on a 25 cm x 4.6 mm ODS-AQ (YMC, Inc.) Column using an acetonitrile:methanol:buffer mobile phase (16:10:74, by volume) at a flow rate of 1.0 mL/min. The mobile-phase buffer was comprised of 0.05M potassium phosphate with 0.01M tetramethylammonium perchlorate, adjusted to pH 6.0 prior to being combined with the organic fractions. Electrochemical detection in the oxidative mode (DET1 = +0.4V, DET2 = +0.85V) was used for quantitation of the analytes.

The drug-plasma concentration of each sample was calculated by least squares linear regression analysis of the peak-area ratio (parent/internal standard) of the spiked plasma standards versus concentration. The assay was linear over the concentration range 1-533 ng/mL, with a mean percent deviation of <6.5% for the analysis of triplicate standards at seven separate concentrations. The maximum plasma concentration (Cₘₐₓ) and the time to reach the maximum plasma concentrations (Tₘₐₓ) were read directly from the ovserved plasma concentration-time data. The plasma elimination half-life (T_{1/2}) was estimated from the log-linear regression of the terminal plasma concentrations as a function of time after dosing. The area under the plasma concentration-time curve was calculated using the linear trapezoidal rule over the single 72-hour dosing interval (AUC₀₋₇₂).

### C. Evaluation of In-vivo Drug Release

Four experimental sustained-release suspensions were administered to different groups of three beagle dogs each. Each formulation was evaluated using a 2 mg/kg subcutaneous dose. The plasma concentrations were compared to those obtained from an equivalent dose of the immediate-release injection, Torbugesic-SA. The pharmacokinetic parameters, Cₘₐₓ, Tₘₐₓ, T1/2, and AUC₀₋₇₂, are given below in Table 7.

Butorphanol was rapidly absorbed from the immediate-release formulation, with the peak concentration (169.3 ±66.7 nh/mL) recorded one hour after dosing. All four experimental formulations produced a sustained-release profile of butorphanol. The drug-plasma concentration was maintained within the proposed therapeutic window (20-100 ng/mL) over a 24-hour period for the two oil suspensions and the 5 mg/mL aqueous suspension. Also, the time that maximum concentration is reached was relatively fast for all sustained-release formulations. For subcutaneous administration of the aqueous suspensions, the Tₘₐₓ was 1.5 and 2.0 hours for the 5 and 10 mg/mL formulations, respectively. A slightly slower absorption was observed for the oil suspensions, with Tₘₐₓ values averaging three and four hours for the 10 and 20 mg/mL formulations, respectively. It was also observed that the highest Cₘₐₓ and AUC were associated with the 5 mg/mL aqueous suspension.

**Table 7.**

| Pharmacokinetic Parameter of the Five Formulations | | | | |
|---|---|---|---|---|
| Formulation | | Cₘₐₓ (ng/mL) | Tₘₐₓ (hr) | AUC₀₋₇₂ (ng•hr/mL) |
| 5 mg/mL Aqueous Suspension | Mean (SEM) | 99.7 (39.8) | 1.5 (0.5) | 1068.9 (231.5) |
| 10 mg/mL Aqueous Suspension | Mean (SEM) | 17.3 (2.9) | 2.0 (0.0) | 632.0 (101.4) |
| 10 mg/mL Oil Suspension | Mean (SEM) | 48.2 (1.0) | 3.0 (1.5) | 923.8 (134.5) |
| 20 mg/mL Oil Suspension | Mean (SEM) | 33.3 (7.5) | 4.0 (0.0) | 698.1 (28.4) |
| 2 mg/mL Torbugesic-SA | Mean (SEM) | 169.3 (38.5) | 1.0 (0.0) | 1537.5 (282.5) |

The data for two oil suspensions and the 5 mg/ml aqueous suspension show a sustained drug-release profile with the drug-plasma concentration maintained within the therapeutic range of 20-80 ng/mL over a 24-hour period of time.

## Claims

1. A pharmaceutical composition for the controlled release of butorphanol comprising an effective therapeutic amount of butorphanol free base suspended in an aqueous buffer having a pH less than 7.0.

2. The composition of claim 1 wherein the butorphanol is in the form of microparticles having an average diameter from 5 to 25 microns.

3. The composition of claim 1 having a pH from 6.2 to 6.5.

4. The composition of claim 3 having a pH from 6.2 to 6.3.

5. The composition of claim 1 wherein butorphanol is present in a concentration from 4 to 10 mg/ml.

6. The composition of claim 5 wherein butorphanol is present in a concentration from 5 to 7.5 mg/ml.

7. The composition of claim 1 further comprising an effective amount of a non-ionic solubilizing agent.

8. The composition of claim 7 wherein the solubilizing agent is a polyoxyethylene sorbitan fatty acid ester.

9. The composition of claim 8 wherein the polyoxyethylene sorbitan fatty acid ester is polysorbate 80.

10. The composition of claim 1 further comprising an effective amount of a preservative.

11. The composition of claim 10 wherein the preservative is an alkylparaben.

12. The composition of claim 11 wherein the alkylparaben is methylparaben or propylparaben.

13. A pharmaceutical composition for the controlled release of butorphanol comprising an effective therapeutic amount of a butorphanol salt suspended in oil.

14. The composition of claim 13 wherein the butorphanol salt is butorphanol tartrate.

15. The composition of claim 13 wherein butorphanol is present in a concentration from 5 to 30 mg/ml.

16. The composition of claim 15 wherein butorphanol is present in a concentration from 10 to 20 mg/ml.

17. The composition of claim 13 wherein the oil is cottonseed oil, corn oil, peanut oil, sesame oil or soybean oil.

18. The composition of claim 17 wherein the oil is soybean oil.

19. The composition of claim 13 further comprising an effective amount of a suspending agent.

20. The composition of claim 19 wherein the suspending agent is a sorbitan fatty acid ester.

21. The composition of claim 20 wherein the sorbitan fatty acid ester is Span 85.

22. Use of the composition of claim 1 for manufacturing a preparation for providing effective pain management over a period of time of 12 to 24 hours to a human in need of such management.

23. The use of claim 22 wherein the period of time is 18 to 24 hours.

24. The use of claim 22 wherein the effective amount is that amount sufficient to maintain plasma butorphanol concentration at a level of between 20 and 100 ng/ml for a period of time of 12 to 24 hours.

25. Use of the composition of claim 14 for manufacturing an injectable preparation for providing effective pain management over a period of 12 to 24 hours to a human in need of such management.

26. The use of claim 25 wherein the period of time is 18 to 24 hours.

27. The use of claim 25 wherein the effective amount is that amount sufficient to maintain plasma butorphanol concentration at a level of between 20 and 100 ng/ml for a period of time of 12 to 24 hours.

## Patentansprüche

1. Eine pharmazeutische Zusammensetzung für die kontrollierte Freisetzung von Butorphanol, die eine wirksame therapeutische Menge von Butorphanol freie Base umfaßt, suspendiert in einem wässerigen Puffer, der einen pH von weniger als 7.0 hat.

2. Die Zusammensetzung von Anspruch 1, worin das Butorphanol in der Form von Mikropartikeln ist, die einen durchschnittlichen Durchmesser von 5 bis 25 Mikrons haben.

3. Die Zusammensetzung von Anspruch 1, die einen pH von 6,2 bis 6,5 hat.

4. Die Zusammensetzung von Anspruch 3, die einen pH von 6,2 bis 6,3 hat.

5. Die Zusammensetzung von Anspruch 1, worin Butorphanol in einer Konzentration von 4 bis 10 mg/ml anwesend ist.

6. Die Zusammensetzung von Anspruch 5, worin Butorphanol in einer Konzentration von 5 bis 7,5 mg/ml anwesend ist.

7. Die Zusammensetzung von Anspruch 1, die weiterhin eine wirksame Menge eines nicht ionischen Lösungsvermittlers umfaßt.

8. Die Zusammensetzung von Anspruch 7, worin der Lösungsvermittler ein Polyoxyethylensorbitanfettsäurester ist.

9. Die Zusammensetzung von Anspruch 8, worin der Polyoxyethylensorbitanfettsäurester Polysorbat 80 ist.

10. Die Zusammensetzung von Anspruch 1, die weiterhin eine wirksame Menge eines Konservierungsstoffes umfaßt.

11. Die Zusammensetzung von Anspruch 10, worin der Konservierungsstoff ein Alkylparaben ist.

12. Die Zusammensetzung von Anspruch 11, worin das Alkylparaben Methylparaben oder Propylparaben ist.

13. Eine pharmazeutische Zusammensetzung für die kontrollierte Freisetzung von Butorphanol, die eine wirksame therapeutische Menge eines Butorphanolsalzes, suspendiert in Öl, umfaßt.

14. Die Zusammensetzung von Anspruch 13, worin das Butorphanolsalz Butorphanoltartrat ist.

15. Die Zusammensetzung von Anspruch 13, worin Butorphanol in einer Konzentration von 5 bis 30 mg/ml anwesend ist.

16. Die Zusammensetzung von Anspruch 15, worin Butorphanol in einer Konzentration von 10 bis 20 mg/ml anwesend ist.

17. Die Zusammensetzung von Anspruch 13, worin das Öl Baumwollsamenöl, Maiskeimöl, Erdnußöl, Sesamöl oder Sojabohnenöl ist.

18. Die Zusammensetzung von Anspruch 17, worin das Öl Sojabohnenöl ist.

19. Die Zusammensetzung von Anspruch 13, die weiterhin eine wirksame Menge eines Suspendiermittels umfaßt.

20. Die Zusammensetzung von . Anspruch 19, worin das Suspendiermittel ein Sorbitanfettsäureester ist.

21. Die Zusammensetzung von Anspruch 20, worin der Sorbitanfettsäureester Span 85 ist.

22. Verwendung der Zusammensetzung von Anspruch 1 zur Herstellung einer Zubereitung zur Bereitstellung einer wirksamen Schmerzbehandlung über einen Zeitraum von 12 bis 24 Stunden für einen Menschen, der eine solche Behandlung benötigt.

23. Die Verwendung von Anspruch 22, worin der Zeitraum 18 bis 24 Stunden ist.

24. Die Verwendung von Anspruch 22, worin die wirksame Menge jene Menge ist, die ausreicht, um die Plasmabutorphanolkonzentration bei einem Pegel von zwischen 20 und 100 ng/ml für einen Zeitraum von 12 bis 24 Stunden aufrechtzuerhalten.

25. Verwendung der Zusammensetzung von Anspruch 14 zur Herstellung einer injizierbaren Zubereitung zum Bereitstellen einer wirksamen Schmerzbehandlung über einen Zeitraum von 12 bis 24 Stunden für einen Menschen, der eine solche Behandlung benötigt.

26. Die Verwendung von Anspruch 25, worin der Zeitraum von 18 bis 24 Stunden ist.

27. Die Verwendung von Anspruch 25, worin die wirksame Menge jene Menge ist, die ausreicht, um die Plasmabutorphanolkonzentration bei einem. Pegel von zwischen 20 und 100 ng/ml für einen Zeitraum von 12 bis 24 Stunden aufrechtzuerhalten.

## Revendications

1. Composition pharmaceutique pour la libération contrôlée de butorphanol comprenant une quantité thérapeutique efficace de base libre de butorphanol en suspension dans un tampon aqueux ayant un pH inférieur à 7,0.

2. Composition selon la revendication 1, dans laquelle le butorphanol est sous la forme de microparticules ayant un diamètre moyen de 5 à 25 microns.

3. Composition selon la revendication 1 ayant un pH de 6,2 à 6,5.

4. Composition selon la revendication 3 ayant un pH de 6,2 à 6,3.

5. Composition selon la revendication 1, dans laquelle le butorphanol est présent dans une concentration de 4 à 10 mg/ml.

6. Composition selon la revendication 5, dans laquelle le butorphanol est présent dans une concentration de 5 à 7,5 mg/ml.

7. Composition selon la revendication 1 comprenant en outre une quantité efficace d'un agent solubilisant non ionique.

8. Composition selon la revendication 7, dans laquelle l'agent solubilisant est un ester d'acide gras de polyoxyéthylène sorbitan.

9. Composition selon la revendication 8, dans laquelle l'ester d'acide gras de polyoxyéthylène sorbitan est le polysorbate 80.

10. Composition selon la revendication 1 comprenant en outre une quantité efficace d'un conservateur.

11. Composition selon la revendication 10, dans laquelle le conservateur est un parabène d'alkyle.

12. Composition selon la revendication 11, dans laquelle le parabène d'alkyle est le parabène de méthyle ou le parabène de propyle.

13. Composition pharmaceutique pour la libération contrôlée de butorphanol comprenant une quantité thérapeutique efficace d'un sel de butorphanol en suspension dans de l'huile.

14. Composition selon la revendication 13, dans laquelle le sel de butorphanol est le tartrate de butorphanol.

15. Composition selon la revendication 13, dans laquelle le butorphanol est présent dans une concentration de 5 à 30 mg/ml.

16. Composition selon la revendication 15, dans laquelle le butorphanol est présent dans une concentration de 10 à 20 mg/ml.

17. Composition selon la revendication 13, dans laquelle l'huile est l'huile de coton, l'huile de mais, l'huile d'arachide, l'huile de sésame ou l'huile de soja.

18. Composition selon la revendication 17, dans laquelle l'huile est l'huile de soja.

19. Composition selon la revendication 13 comprenant en outre une quantité efficace d'un agent de suspension.

20. Composition selon la revendication 19, dans laquelle l'agent de suspension est un ester d'acide gras de sorbitan.

21. Composition selon la revendication 20, dans laquelle l'ester d'acide gras de sorbitan est le Span 85.

22. Utilisation de la composition selon la revendication 1 pour la fabrication d'une préparation fournissant un traitement efficace de la douleur sur une période de temps de 12 à 24 heures à un homme ayant besoin d'un tel traitement.

23. Utilisation selon la revendication 22, dans laquelle la période de temps est 18 à 24 heures.

24. Utilisation selon la revendication 22, dans laquelle la quantité efficace est cette quantité suffisante pour maintenir la concentration plasmatique de butorphanol à un niveau compris entre 20 et 100 ng/ml pendant une période de temps de 12 à 24 heures.

25. Utilisation de la composition selon la revendication 14 pour la fabrication d'une préparation injectable fournissant un traitement efficace de la douleur sur une période de 12 à 24 heures à un homme ayant besoin d'un tel traitement.

26. Utilisation selon la revendication 25, dans laquelle la période de temps est 18 à 24 heures.

27. Utilisation selon la revendication 25, dans laquelle la quantité efficace est cette quantité suffisante pour maintenir la concentration plasmatique de butorphanol à un taux compris entre 20 et 100 ng/ml pendant une période de temps de 12 à 24 heures.
